# EUROPEAN PATENT APPLICATION

(11) **EP 4 089 684 A1**
(43) Date of publication of application: **16.11.2022**
(21) Application number: 22172233.3
(22) Date of filing: 09.05.2022
(51) Int. Cl.: G16H 10/40

(54) **PREDICTING GUT MICROBIOME DIVERSITY**

(30) Priority: 12.05.2021 US 202163201777 P
(71) Applicant: ZOE Limited, London SE1 7RW (GB)
(72) Inventor: WOLF, Jonathan Thomas, London, SE1 7RW (GB); DAVIES, Richard James, London, SE1 7RW (GB)
(74) Representative: Patentgruppen A/S

(57) **Abstract**

Techniques are disclosed herein for generating predictions of gut microbiome diversity for a user. In some examples, a nutritional service may utilize data associated with a gut transit time and user data to generate a prediction of gut microbiome diversity for a user. For example, the nutritional service may perform an analysis of the data associated with gut transit time and the answers to questions to generate the prediction. In some examples, the nutritional service identifies a uniqueness of the microbiome, identify interesting species, and the like. The information determined and/or otherwise generated by the nutritional service may be presented to the user on a display.

## Description

This application claims priority to U.S. Provisional Application No. 63/201,777, filed on May 12, 2021, the entire contents of which are incorporated herein by reference.

### Background

Determining healthy food choices for an individual can be challenging. Not only do individuals have a large variety of food choices, but food that is healthy for one individual may not be healthy for another individual. Age, sex, weight, the microbiome of an individual, as well as other factors affect what foods an individual should select to eat. For example, while low carbohydrate food or low-fat food may be beneficial for one individual, that same low carbohydrate or low-fat food choice may not be beneficial for another individual. In many cases, an individual may try many different types of foods and nutritional plans in an attempt to find the nutritional plan and foods that work best for them. Providing guidance about what to eat and how to change their diet can be very difficult and confusing for an individual given the plethora of food choices and the complex nature of the responses the individual has to different foods.

### Summary of the invention

The inventors have identified the above-mentioned problems and challenges related to individual nutrition response, and subsequently made the below-described invention relating to generation of personalized classification of microbiome, which may be used for example as an informative marker of gut function, predict postprandial lipid and glucose responses and visceral fat, guidance or recommendations on food that has not yet been eaten, generate personalized food guidance that takes into account the user's predicted gut microbiome diversity, and more as disclosed below, as well as combinations of these advantages.

The invention relates to a method comprising accessing gut transit time data associated with a user; accessing user data associated with the user; generating personalized classification of microbiome based at least in part on the gut transit time data and the user data, wherein the personalized classification of microbiome are personalized for the user; and causing data associated with the personalized classification of microbiome to be presented within a user interface to the user.

The user may comprise a first user, the gut transit time data comprises first gut transit time data, and the user data comprises first user data, the method further comprising: identifying a second user based at least in part on one of: a characteristic of the first user gut transit time data; first user data, an equivalent characteristic of second gut transit time data associated with the second user; or second user data associated with the second user is within a threshold time to the first user data; accessing microbiome data associated with the second user; and generating the personalized classification of microbiome based at least in part on the microbiome data associated with the second user.

The gut transit time data may be received via an application stored on a device associated with the user, the gut transit time data indicating an amount of gut transit time it takes from consuming an object to expelling the object.

The amount of gut transit time may be associated with a classification based at least in part on a distribution of gut transit times in a population.

The user data may include at least one of age, height, weight, sex assigned at birth, gender identification, location information, diet information, stool information, pet information, or allergy information.

In an embodiment, the method further comprises accessing nutrition data associated with the user; and generating the personalized classification of microbiome based at least in part on the nutrition data.

The nutrition data may be associated with at least one previously eaten food item and may include at least one of a frequency of consumption, a quantity of consumption, grams of fiber, level of food item processing, type of fiber, grams of carbohydrate, glycemic index, sugar content, salt content, alcohol content, whether the food item is fermented, whether the food item is a high polyphenol food item, whether the food item is meat, the composition and source of fat in the food item, a composition of fiber in the food item, a production method, preparation methods applied by the user, an inclusion of flavor enhancers, an inclusion of emulsifiers, a method by which the food item was preserved, a location where the food item was produced, a method by which animals contributing to the food item were reared, a method by which animals contributing to the food item were caught, or a method by which animals contributing to the food item were slaughtered.

In an embodiment the method further comprises receiving at least one input identifying at least one food item; determining food data associated with the at least one food item; determining health data associated with the user based at least in part on the gut transit time data; generating a postprandial response prediction associated with the at least one food item based at least in part on the food data and the health data; and associating at least one biomarker score with the at least one food item based at least in part on the postprandial response prediction.

In an embodiment the method further comprises generating, based at least in part on the gut transit time data and the user data, a recommendation to the user for improving microbiome diversity, increasing good microbes, or decreasing bad microbes; and causing data associated with the recommendation to be presented within the user interface to the user.

The recommendation may include at least one of an eating habit recommendation, a hydration recommendation, or a food item consumption recommendation.

In an embodiment, the method, further comprises receiving additional gut transit time data associated with additional users; determining an average gut transit time data associated with the additional gut transit time data; and causing data associated with a comparison of the average gut transit time data to the gut transit time data associated with the user to be presented within the user interface to the user.

The personalized classification of microbiome may be generated by a machine learning model. The personalized classification of microbiome includes at least a classification of at least one microbe located within the user, the at least one microbe including at least one of Akkermansia muciniphila, Bacteroides or Alistipes spp.

In an embodiment the method further comprises generating personalized classification of microbiome based on accessing at least one of a family health history associated with the user, measures of blood chemistry taken in a fasting state associated with the user, or measures of blood chemistry taken in a postprandial state associated with the user. In an embodiment the method further comprises generating a quality score associated with the personalized classification of microbiome indicating an accuracy level of the personalized classification of microbiome.

The invention relates to a system comprising one or more processors; and a computer program comprising instructions that, when executed by the one or more processors, cause the one or more processors to perform the method of any of the above.

The invention relates to a system comprising one or more processors; and one or more non-transitory computer-readable media comprising instructions that, when executed by the one or more processors, cause the one or more processors to perform operations comprising: accessing gut transit time data associated with a user; accessing user data associated with the user; generating personalized classification of microbiome based at least in part on the gut transit time data and the user data, wherein the personalized classification of microbiome are personalized for the user; and causing data associated with the personalized classification of microbiome to be presented within a user interface to the user.

In an embodiment the system comprises receiving additional gut transit time data associated with additional users; determining an average gut transit time data associated with the additional gut transit time data; and causing data associated with a comparison of the average gut transit time data to the gut transit time data associated with the user to be presented within the user interface to the user.

In an embodiment the user comprises a first user, the gut transit time data comprises first gut transit time data, and the user data comprises first user data, the method further comprising: identifying a second user based at least in part on one of: a characteristic of the first user gut transit time data; first user data, an equivalent characteristic of second gut transit time data associated with the second user; or second user data associated with the second user has at least one characteristics present in to the first user data; accessing microbiome data associated with the second user; and generating the personalized classification of microbiome based at least in part on the microbiome data associated with the second user.

The gut transit time data may be received via an application stored on a device associated with the user, the gut transit time data indicating an amount of gut transit time it takes from consuming an object to expelling the object.

The amount of gut transit time is associated with a classification based at least in part on a distribution of gut transit times in a population.

The gut transit time data is received via an application stored on a device associated with the user, the gut transit time data indicating an amount of gut transit time it takes from consuming an object to expelling the object.

The amount of gut transit time is associated with a classification based at least in part on a distribution of gut transit times in a population.

The invention relates to a method comprising: receiving test data associated with measurement of gut transit time; generating personalized microbiome data based at least in part on the test data, wherein the personalized microbiome data is personalized for a user; and causing data associated with the personalized microbiome data to be presented within a user interface to the user. In connection with the receiving test data, the method may also comprise receiving at least one input identifying at least one food item to be used in measurement of gut transit time for a user.

The personalized microbiome data may be generated by a machine learning model. The personalized microbiome data includes at least a classification of at least one microbe located within the user, the at least one microbe including at least one of Akkermansia muciniphila, Bacteroides or Alistipes spp.

In an embodiment the method further comprises generating personalized microbiome data based on accessing at least one of a family health history associated with the user, measures of blood chemistry taken in a fasting state associated with the user, or measures of blood chemistry taken in a postprandial state associated with the user. In an embodiment the method further comprises generating a quality score associated with the personalized microbiome data indicating an accuracy level of the personalized microbiome data.

### Brief description of the drawings

FIG. 1 is a block diagram depicting an illustrative operating environment in which predictions of gut microbiome diversity using gut transit time are generated.
FIG. 2 is a block diagram depicting an illustrative operating environment in which personalized health tips/food guidance is generated.
FIG. 3 is a block diagram depicting an illustrative operating environment in which a data ingestion service receives, and processes data associated with gut transit time and nutritional responses.
FIG. 4 is a computer architecture diagram showing one illustrative computer hardware architecture for implementing a computing device that might be utilized to implement aspects of the various examples presented herein.
FIG. 5 is a flow diagram showing a process illustrating aspects of a mechanism disclosed herein for generating predictions of gut microbiome diversity for a user.
FIG. 6 is a flow diagram showing a process illustrating aspects of a mechanism disclosed herein for obtaining test data, including gut transit time data, that may be utilized for generating microbiome diversity for users.
FIG. 7 is an exemplary diagram illustrating one or more user interfaces for obtaining gut transit data, and other data related to predicting diversity of gut microbiome.
FIG. 8 is an exemplary diagram illustrating one or more user interfaces for obtaining user data, and other data related to predicting diversity of gut microbiome.
FIG. 9 is an exemplary diagram illustrating one or more user interfaces for obtaining food, and other data related to predicting diversity of gut microbiome.
FIG. 10 is an exemplary diagram illustrating one or more user interfaces for presenting gut transit data, and other data related to predicting diversity of gut microbiome.
FIG. 11 is an exemplary diagram illustrating one or more user interfaces for presenting gut transit data, and other data related to predicting diversity of gut microbiome.
FIG. 12 is an exemplary diagram illustrating one or more user interfaces for presenting gut transit data, and other data related to predicting diversity of gut microbiome.
FIG. 13 is an exemplary diagram illustrating one or more user interfaces for presenting match data, and other data related to predicting diversity of gut microbiome.
FIG. 14 is an exemplary diagram illustrating one or more user interfaces for presenting match data, and other data related to predicting diversity of gut microbiome.
FIG. 15 is an exemplary diagram illustrating one or more user interfaces for presenting match data, and other data related to predicting diversity of gut microbiome.
FIG. 16 is an exemplary diagram illustrating one or more user interfaces for presenting microbiome data, and other data related to predicting diversity of gut microbiome.
FIG. 17 is an exemplary diagram illustrating one or more user interfaces for presenting microbiome data, and other data related to predicting diversity of gut microbiome.
FIG. 18 is an exemplary diagram illustrating one or more user interfaces for presenting microbiome data, and other data related to predicting diversity of gut microbiome.
FIG. 19 is an exemplary diagram illustrating one or more user interfaces for presenting microbiome data, and other data related to predicting diversity of gut microbiome.
FIG. 20 is an exemplary diagram illustrating one or more user interfaces for presenting gut transit time data, and other data related to predicting diversity of gut microbiome.
FIG. 21 is an exemplary diagram illustrating one or more user interfaces for presenting gut transit time data, and other data related to predicting diversity of gut microbiome.
FIG. 22 is an exemplary diagram illustrating one or more user interfaces for presenting gut transit time data, and other data related to predicting diversity of gut microbiome.
FIG. 23 is an exemplary diagram illustrating one or more user interfaces for presenting recommendation data, and other data related to predicting diversity of gut microbiome.
FIG. 24 is an exemplary diagram illustrating one or more user interfaces for presenting recommendation data, and other data related to predicting diversity of gut microbiome.

### Detailed description

The following detailed description is directed to system and methods for generating predictions of gut microbiome diversity for users. Using technologies described herein, gut transit time may be used to predict the diversity of gut microbiome for a user. In some examples, a user measures a gut transit time. The "gut transit time" is the time it takes from consuming an object to expelling the object. For instance, the gut transit time may be the time between ingestion of a food including food coloring paste, such as royal blue (or some other color), and the first time the user sees the appearance of the color of the food coloring in their stool. According to some configurations, a user eats food, such as a muffin, that has been died "blue," such that it is easily identified by its color when it appears in the stool of the user.

In some examples, the prediction is also based on answers to questions that the user has provided. For instance, the user may answer questions, such as but not limited to age, height, weight, gender, location information, diet information, pet information, allergy information, and the like

According to some configurations, a nutritional service may utilize the data associated with the gut transit time and the questions to generate the prediction of the gut microbiome diversity for a user. For example, the nutritional service may use a machine learning mechanism to perform an analysis of the data associated with gut transit time and the answers to questions to generate the prediction. In some examples, the nutritional service identifies a uniqueness of the microbiome, identify interesting species, and the like. According to some configurations, the microbiome data may include gut transit time.

In some examples, the gut transit time data of the user is utilized with other data that is gathered about the user, as well as other users. For instance, users may provide responses to questionnaires, data about gut transit times of different users, data about food that is eaten, location information, sleep habits, and the like.

In some examples, the nutritional service may provide a user interface (UI), such as a Graphical User Interface (GUI) for a user to view and interact with data and/or other data associated with the prediction of gut microbiome diversity. For instance, the GUI may display a gut transit time for the user, a comparison to other user(s), personalized health tips, a classification of the gut microbiome diversity, and the like. The nutritional service may also provide recommendation data that identifies one or more recommendations relating to changing the microbiome of the user, and the like. In some examples, the UI/GUI may display information relating to determining a time a food was consumed to the time the user first saw the appearance of the color (e.g., blue) within a stool. In some examples, the date and time (e.g., hours, minutes, etc.) of the blue appearance is automatically recorded within a mobile application after a user selected a UI element (e.g., clicked on the associated UI button). The UI/GUI may also provide UI elements for obtaining information about the user (e.g., height, weight, diet, etc.)

In some examples, personalized food guidance may be provided that takes into account an individual's personalized responses to foods, such as the individual's glucose and fat responses, the individual's microbiome, the individual's overall health, potential health risks for the individual, and/or other data associated with the individual. Providing an individual with personalized food guidance can make choosing food easier and healthier and help them to understand how to improve their existing diet.

According to some examples, the personalized food guidance may include guidance data such as but not limited to predicted gut microbiome diversity, personalized health scores (e.g., glucose, fat, cholesterol, etc.), guidance/recommendations on food that has not yet been eaten, and/or other guidance tips/data (e.g., via a User Interface (UI))). The guidance data may be used by an individual to help them understand how they can improve their diet. For instance, a user may receive guidance data by viewing a UI on a mobile device, or some other device. The guidance data may also indicate a score, or some other indicator, that summarizes information about how the user has eaten over a particular time period (e.g., a day, a week, and the like).

To generate personalized food guidance, the nutritional service can produce personalized score(s) for one or more personalized health scores, and/or foods/meals that is based on personalized data about an individual and the individual's biological responses to the particular food/meals. The personalized data may include data that predicts how one or more of the individual's biomarkers will change postprandially, data that predicts an individual's potential risks such as diabetes or cardiovascular disease, as well other data associated with the individual. The nutritional service may combine multiple data sources about the individual's response to each food in such a way that the nutritional service takes into account the individual's own personalized risks, so as to optimize the overall meal score for each individual based upon their own body's responses.

In some examples, the nutritional service may use one or more other services, to score the individual's responses to food on more than one dimension (e.g., the impact of the food on biomarkers such as glucose, triglycerides, insulin, energy and hunger and its impact on shifting the microbiome towards a specific goal). In some cases, these scores may depend on factors that change rapidly such as the time of day, what was eaten previously or the amount of sleep.

The nutritional service may also use other data when generating the scores for a particular food. For instance, the nutritional service may use properties of the food that can be measured independently of the user, such as grams of fiber, level of food processing, type of fiber, glycemic index, sugar content, salt content, alcohol content, whether the food is fermented, whether the food is a high polyphenol food such as dark chocolate or vegetables, whether the food is meat, the composition and source of fat in the food, the composition of the fiber in the food, the production method, preparation methods applied by the user (such as cooking method), the inclusion of particular ingredients such as (but not limited to) flavor enhancers or emulsifiers, the method by which the food was preserved, the location where the food was produced, the method by which animals contributing to the product were reared, caught or slaughtered, and the like. The nutritional service may weight these properties differently depending upon the individual (for example people with high lipid responses may be guided more strongly against alcohol and in favor of fiber).

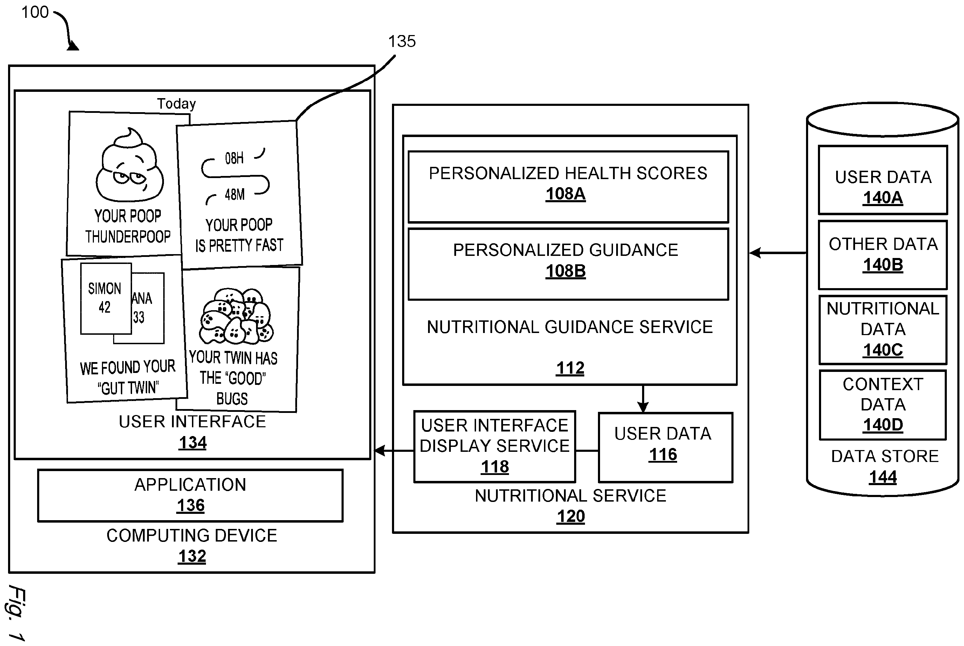

FIG. 1 is a block diagram depicting an illustrative operating environment 100 in which a diversity of a gut microbiome diversity is predicted. As illustrated, environment 100 includes a nutritional service 120, a nutritional guidance service 112, a UI display service 118, a data store 144, and a computing device 132.

As illustrated in FIG. 1, the operating environment 100 includes one or more computing devices 132 in communication with a nutritional service 120. In some examples, the environment 100 may be associated with and/or implemented by resources provided by a service provider network such as provided by a cloud computing company. The environment 100 may include a collection of computing resources (e.g., computing devices such as servers). The computing resources may include a number of computing, networking and storage devices in communication with one another. In some examples, the computing resources may correspond to physical computing devices and/or virtual computing devices implemented by one or more physical computing devices.

According to some examples, the UI 134 may display information to a user relating to gut transit time. As illustrated, the UI 134 is presenting information 135 to the user that classifies the gut transit time of the user (e.g., "thunderpoop"), a gut transit time, an indication of how the gut transit time compares to other users, a "gut twin" that indicates a similar user based on the predicted diversity of gut microbiome, and microbe information.

In some examples, the UI 134 is also used when determining the gut transit time and answering questions that can be used to generate the prediction of gut microbiome diversity. For instance, the UI 134 may be used to determine a time a food was consumed that included food coloring, (or some other marker) to the time the user first saw the appearance of the color (e.g., blue) within an excreted stool. For example, the food may be injected with a blue dye (or possibly another color), which may be referred to herein as the "blue dye method" before being consumed by a user. Using the blue dye method provides benefits over other transit time measures (e.g., scintigraphy, wireless motility capsule, radiopaque markers, and breath testing) since it is inexpensive and scalable. For example, specialized equipment and staff are not required for the blue dye method. Further, gut transit time, measured via the blue dye method, is a more informative marker of gut microbiome function than traditional measures of stool consistency and frequency.

In some examples, the date and time (hours, minutes) of the appearance of the coloring (or other marker) is automatically recorded by the nutritional service 120, or some other device or component, after a user selected a UI element (e.g., clicked on the associated UI button) or some other triggering event (e.g., recognition within image data of the color of the dye within the stool). In some configurations, the user or some other device/component (e.g., image processing) may also identify/record stool frequency and/or consistency. For instance, the user, or some device/component, may record how often they have a bowel movement and record the consistency (e.g., using the Bristol Stool Form (BSF) scale). Generally, a lower BSF scale score corresponds to longer gut transit time.

The nutritional service 120 may use the gut transit time to generate a classification for the diversity of the microbiome for the user. According to some examples, the nutritional service 120 may select from different classifications (e.g., two, three, four, ...) based on the gut transit time for the user. For instance, the nutritional service 120 may select from a number of different classifications based at least in part on a distribution of gut transit times in a population (e.g., a fastest classification of less than 14 hours, a normal classification between 14 hours and 58 hours, a fast normal classification between 14 hours and 38 hours, a slower normal classification between 38 hours and 58 hours, and a slow classification of greater than 58 hours) depending on the gut transit time for the user. More or less classifications may be used. Gut microbiome composition predicts gut transit time classes and longer gut transit time are linked with Akkermansia muciniphila, Bacteroides and Alistipes spp relative abundances.

The gut transit time may provide a good indication of variation in the gut microbiome, in terms of both relative abundance and alpha diversity. Alpha diversity reflects how diverse a single microbiome sample is, using a measure of richness (i.e., number of species detected) and the Shannon index, which accounts for both evenness and abundance. As such, the classification for the user based on a gut transit time may be used as an informative marker of gut function and may be used to predict postprandial lipid and glucose responses and visceral fat.

The data store 144 may include a variety of data 140, such as user data 140A, other data 140B, nutritional data 140C, and/or context data 140D. In some configurations, an individual (e.g., an individual associated with the computing device 132) may generate and provide user data 140A, such as health data 202 and test data 205) using a variety of at home biological collection devices, which collect a biological sample which requires a biological assay to be performed to generate the user data 140A. Some of the user data 140A may be biomarker data, such as blood glucose results collected by a continuous glucose monitor (CGM), as well as other types of data. In some cases, the other data 140B may include non-biomarker data such as photos, time stamps, as well as other data associated with a user or group of users.

Some of this data 140 may be nutritional data associated with individual foods, such as nutritional data 140C. In some configurations this may include data from nutritional databases and may include nutritional information such as fiber, amount of sugar, vitamin composition, vitamin amount, fat composition, fat amount, and the like. Some of this data may come from individuals who have recorded eating such food and shared their own biological responses to this food such as blood glucose responses or reported hunger. By aggregating this data and using other information about these individuals, in some configurations some of the nutritional data for this food such as its impact on blood sugar can be calculated.

Some of this data 140 may be context data associated with a context in which food was consumed, such as context data 140D. For example, the context data 140D may include information received from the individual consuming the food items (and/or another individual on behalf of the individual consuming the food items) and may include a time of day of consumption, a previously eaten food item, and/or an amount of sleep associated with the individual.

Some of this data 140 may be gut transit time data associated with a gut transit time of a user, such as transit time data 140E. For example, the transit time data 140E may indicate the time it takes from consuming an object to expelling the object. For instance, the gut transit time may be the time between ingestion of a food including food coloring paste, such as royal blue (or some other color), and the first time the user sees the appearance of the color of the food coloring in their stool. According to some configurations, a user eats food, such as a muffin, that has been died "blue," such that it is easily identified by its color when it appears in the stool of the user.

According to some configurations, a nutritional service 120 may utilize the transit time data 140E to generate the prediction of the gut microbiome diversity for a user. For example, the nutritional service 120 may use a machine learning mechanism to perform an analysis of the transit time data 140E to generate the prediction. In some examples, the nutritional service 120 identifies a uniqueness of the microbiome, identify interesting species, and the like. According to some configurations, the microbiome data may include gut transit time.

An individual may also provide data 140 using computing device 132. In some configurations an individual can input data 140 into one or more software applications 136. For example, an individual may enter data used to determine gut transit time, the food they consumed, a value indicated by a measurement device, their waist measurement, and the like. Alternatively, and/or in addition to the above, data 140 generated by other measurements can be used to assist in determining when a food was eaten, and/or a test was performed.

The nutritional service 120 can access the data 140 when generating the predictions of gut microbiome diversity for a particular user. In some examples, the nutritional service 120 utilizes both data associated with the user providing the data and data from other users. According to some examples, the data 140 can include data obtained from a clinical setting, which is typically more accurate than at home measurements. In some examples, the nutritional service 120 may utilize one or more machine learning mechanisms. For example, the nutritional service 120 can use a classifier to classify the data within a classification category. In other examples, the nutritional service 120 may generate one or more scores using one or more machine learning mechanisms. The data used by the nutritional service 120 may include data obtained from other users, including questions, gut transit times, microbiome analysis of stool samples in some examples, and the like.

As briefly discussed above, the nutritional service 120 generates personalized data for the user. Instead of providing a user, such as a user of computing device 132 with food guidance that is generalized for a group of individuals, the nutritional service 120 generates personalized food guidance that takes into account the user's predicted gut microbiome diversity.

According to some configurations, the nutritional service 120 uses a nutritional guidance service 112 to generate a variety of personalized food guidance data that may be provided to a user via UI 134, or through some other mechanism. The personalized food guidance data may include data such as but not limited to personalized health scores 108A (e.g., glucose, fat, cholesterol, etc.) and personalized health guidance 108B (e.g., guidance/tips/recommendations for how to improve microbiome health).

In some examples, the nutritional guidance service 112 provides the guidance data (e.g., the personalized health scores 108A and/or the personalized health guidance 108B) via user data 116 to a user interface display service 118 to provide to the user via UI 134. The user data 116 displayed in UI 134 may be used by an individual to help them understand how they can improve their diet. For instance, a user may receive guidance data by viewing UI 134 on a mobile device, or some other device, to determine how to improve the diversity of their gut microbiome.

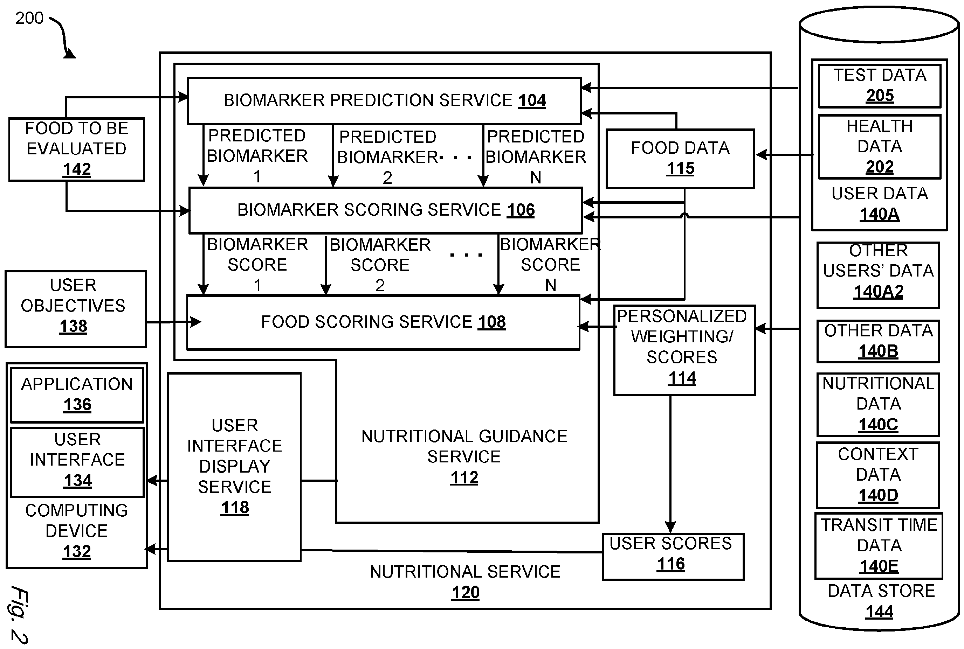

FIG. 2 is a block diagram depicting an illustrative operating environment 200 in which personalized health tips/food guidance is generated. As illustrated, environment 200 includes more details as compared to environment 100. For example, environment 200 includes biomarker prediction service 104, biomarker scoring service 106, food scoring service 108 as part of the nutritional guidance service 112. In other configurations, the services may be located in different environments and/or locations.

In some examples, the nutritional guidance service 112 may receive a single input (e.g., the glucose rise linked to foods). In this example, the nutritional guidance service 112 generates a prediction of the biomarker of interest using biomarker prediction service 104 and a score for that single biomarker using the biomarker scoring service 106. However, in other examples where more than one aspect of the food is calculated, nutritional guidance service 112 predicts many different biomarkers (e.g., biomarkers 1-N) of the assessed aspects (glucose response, blood fat response, and the like), scores each biomarker change individually using the biomarker scoring service 106 and weights the scores so that the scores are comparable and then combined into a single score using food scoring service 108.

In some examples, in addition to utilizing biomarker inputs, the nutritional guidance service 112 also utilizes non-biomarker inputs such as but not limited to food data 115 such as fiber content, amount of processing on the food (e.g., high, medium, low), polyphenol content, fermentation and the like. In some examples the nutritional guidance service uses information about the individual to determine personalizing weighting 114 for combining these scores, which determine how to combine the different biomarker scores 106 and food data, 115 into a single score.

The biomarker prediction service 104 generates a prediction of the postprandial response of the user's biomarker to the food being evaluated. The biomarker scoring service 106 generates scores that are personalized for a particular user. The scores generated by the biomarker scoring service 106 are related to the predicted health impact of these biomarker changes on the particular user, so for example a small rise in blood fat after a meal might have no negative impact on health and so the score for blood fat might be at the best level for that meal but a large rise in blood fat 6 hours after a meal might be detrimental to health and lead to a bad score for blood fat for that meal for that person. The nutritional service 120 may then use these biomarker scores to generate food scores for one or more foods/combination of foods/meals to make individual food recommendations by generating personalized meal scores for any meal using food scoring service 108. In some examples, the food scoring service 108 uses personalized weighting/scores 114 to determine how to weight the different biomarker scores when combining them to generate a single meal score. For example, the food scoring service might need to combine a glucose score, a blood score and a gut health score, and might do so by weighting each of these scores and then combining them.

In some examples, the nutritional service 120 may use one or more other services, to score the individual's responses to food on more than one dimension, such as its impact on shifting the microbiome towards a specific goal, based on user objectives 138. For example, the food scoring service 108 may account for one or more user objectives 138 received by the user, and/or on behalf of the user (e.g., by a health professional), indicating one or more user objectives, such as but not limited to, weight loss, cholesterol decrease, glucose level normalization, blood pressure normalization, etc.

In some examples, if the personalized impact of a food (e.g., food 142) is to be calculated by using predictions of the glucose and triglycerides levels after eating that food combined with the food's impact on the microbiome for that individual, generating a meal score by the food scoring service 108 may consist of using the personalized weighting/scores to combine all three of these scores. The nutritional service 120 is designed to do this such that it recommends the best possible food for the individual based upon the inputs provided. In other examples the nutritional service 120 will use information beyond biomarker predictions as inputs into its biomarker scoring, for example it may take into account family health history, measures of blood chemistry taken in a fasting state such as baseline glucose or triglycerides, or measures of blood chemistry that do not change postprandially such as cholesterol or HbA1c. The nutritional service 120 can also utilize one or more data analysts, machine learning, and/or some other mechanism to generate a quality score for the data.

In some examples, the personalized impact of the food 142 being evaluated and any associated recommendation may be in the form of the user data 116 and provided, via the UI display service 118, to the UI 134 and/or the application 136 of the computing device 132 for presentation to the user.

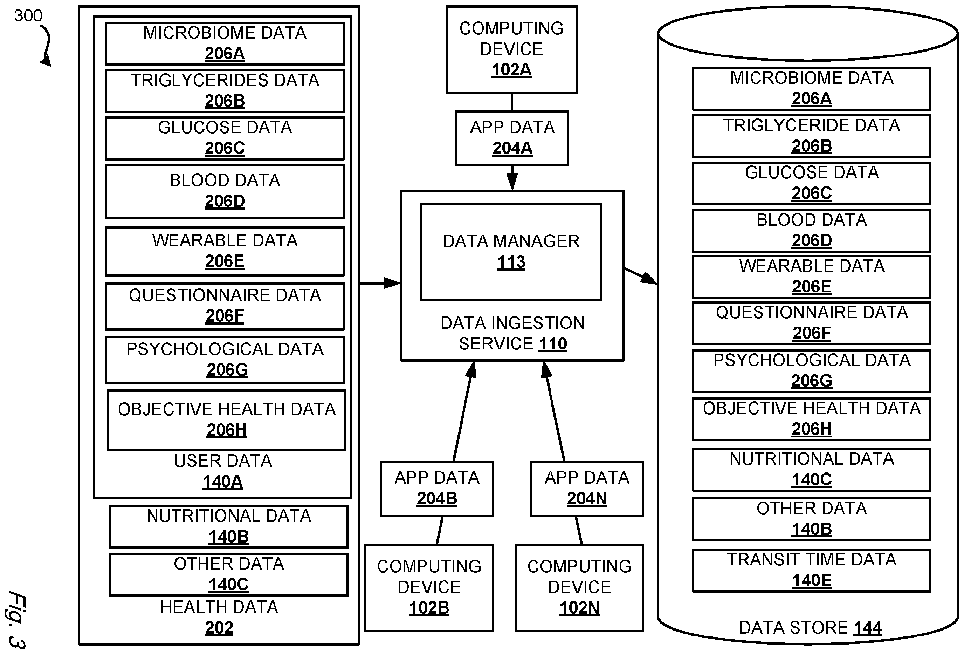

FIG. 3 is a block diagram depicting an illustrative operating environment in which a data ingestion service 110 and a data manager 113 receives, and processes data associated with gut transit time and nutritional responses. The data ingestion service 110 and/or the data manager 113 may be a component and/or a subcomponent of the nutritional service 120 and/or the nutritional guidance service 112. In other examples, the data ingestion service 110 and/or the data manager 113 may be remote component operating within the environment 100 and/or the environment 200.

In some configurations, the data manager 113 is configured to receive data such as, health data 202 that can include, but is not limited to microbiome data and gut transit time data 206A, triglycerides data 206B, glucose data 206C, blood data 206D, wearable data 206E that can include circadian data, questionnaire data 206F, psychological data (e.g., hunger, sleep quality, mood, ...) 206G, objective health data (e.g., height, weight, medical history, ...) 206H, nutritional data 140B, and other data 140C.

According to some examples, the microbiome data 206A includes data about the gut microbiome of an individual. The gut microbiome can host a large number of microbial species (e.g., > 1000) that together have millions of genes.

The triglycerides data 206B may include data about triglycerides for an individual. In some examples, the triglycerides data 206B can be determined from an At Home Blood Test which in some cases is a finger prick on to a dried blood spot card. The glucose data 206C includes data about blood glucose. The glucose data 206C may be determined from various testing mechanisms, including at home measurements, such as a continuous glucose meter.

The blood data 206D may include blood tests relating to a variety of different biomarkers. As discussed above, at least some blood tests can be performed at home. In some configurations, the blood data 206D is associated with measuring blood sugar, insulin, c-peptides, triglycerides, cholesterol, IL-6 inflammation, ketone bodies, nutrient levels, allergy sensitivities, iron levels, blood count levels, HbA1c, and the like.

The wearable data 206E can include any data received from a computing device associated with an individual. For instance, an individual may wear an electronic data collection device 103, such as an activity-monitoring device, that monitors motion, heart rate, heart rate variability, determines how much an individual has slept that may include the types of sleep, the times an individual is awake, the number of calories burned, activities performed, blood pressure, body temperature, and the like. The individual may also wear a continuous glucose meter that monitors blood glucose levels.

The questionnaire data 206F can include data received from one or more questionnaires, and/or surveys received from one or more individuals. The psychological data 206G, that may be subjectively obtained, may include data received from the individual and/or a computing device that generates data or input based on a subjective determination (e.g., the individual states that they are still hungry after a meal, or a device estimates sleep quality based on the movement of the user at night perhaps combined with heart rate data). The objective health data 206H includes data that can be objectively measured, such as but not limited to height, weight, medical history, and the like.

The nutritional data 140B can include data about food, which is referred to herein as "food data". For example, the nutritional data can include nutritional information about different food(s) such as their macronutrients and micronutrients or the bioavailability of its nutrients under different conditions (raw vs cooked, or whole vs ground up).

The other data 140B can include other data associated with the individual. For example, the other data 140B can include data that can be received directly from a computer application that logs information for an individual (e.g., food eaten, sleep, ...) and/or from the user via a user interface.

In some examples, different computing devices 102 associated with different users provide application data 204 to the data manager 113 for ingestion by the data ingestion service 110. As illustrated, computing device 102A provides app data 204A to the data manager 113, computing device 104B provides app data 204B to the data manager 113, and computing device 104N provides app data 204N to the data manager 113. There may be any number of computing devices utilized.

As discussed briefly above, the data manager 113 receives data from different data sources, processes the data when needed (e.g., cleans up the data for storage in a uniform manner), and stores the data within one or more data stores, such as the data store 144.

The data manager 113 can be configured to perform processing on the data before storing the data in the data store 144. For example, the data manager 113 may receive data for ketone bodies and then use that data to generate ketone body ratios. Similarly, the data manager 113 may process food eaten and generate meal calories, number of carbohydrates, fat to carbohydrate ratios, how much fiber consumed during a time period, and the like. The data stored in the data store 144, or some other location, can be utilized by the nutritional service 120 to determine an accuracy of at home measurements of nutritional responses performed by users. The data outputted by the data manager 113 to the nutritional service 120 may therefore contain different values than are stored in the data store 144, for example if a food quantity is adjusted.

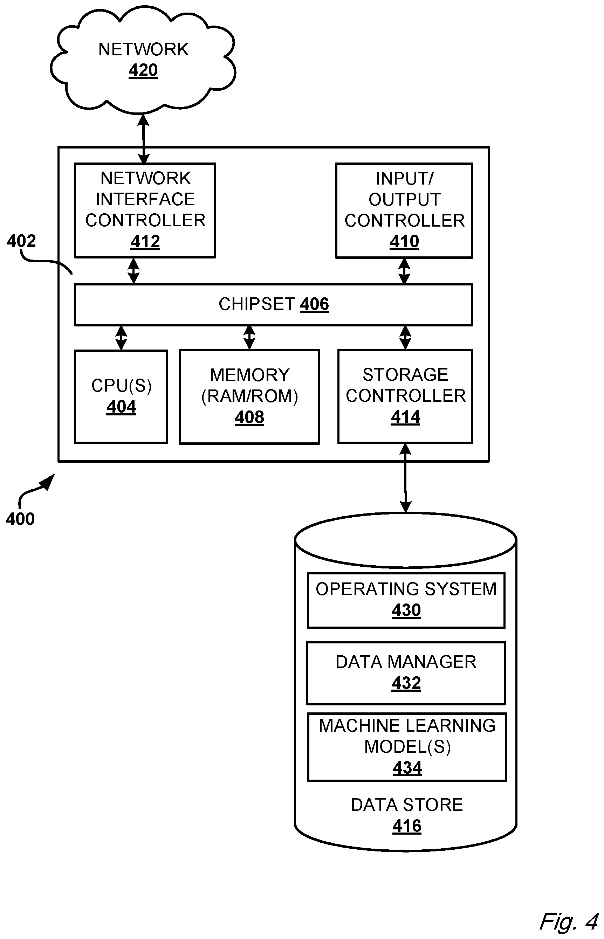

FIG. 4 shows an example computer architecture for a computer 400. The computer architecture shown in FIG. 4 illustrates a conventional server computer, workstation, desktop computer, laptop, mobile device, tablet, network appliance, digital cellular phone, smart watch, or other computing device, and may be utilized to execute any of the software components presented herein. For example, the computer architecture shown in FIG. 4 may be utilized to execute software components for performing operations as described above as described above with regard to the environments 100, 200, and/or 300. The computer architecture shown in FIG. 4 might also be utilized to implement a computing device 132, or any other of the computing systems described herein.

The computer 400 includes a baseboard 402, or "motherboard," to which a multitude of components or devices may be connected by way of a system bus or other electrical communication paths. In one illustrative example, one or more central processing units ("CPUs") 404 operate in conjunction with a chipset 406. The CPUs 404 may be standard programmable processors that perform arithmetic and logical operations necessary for the operation of the computer 400.

The chipset 406 provides an interface between the CPUs 404 and the remainder of the components and devices on the baseboard 402. The chipset 406 may provide an interface to memory 408, such as RAM and/or read-only memory ("ROM"). The memory may store software components utilized for the operation of the computer 400 in accordance with the examples described herein.

The computer 400 may operate in a networked environment using logical connections to remote computing devices and computer systems through a network, such as the network 420. The chipset 406 may include functionality for providing network connectivity through a network interface controller, such as a cellular network adapter, WiFi network adapter, Ethernet adapter, and the like. The NIC 412 is capable of connecting the computer 400 to other computing devices over the network 420.

The computer 400 may be connected to a mass storage device 416 that provides storage for the computer. The mass storage device 416 may store system programs, application programs, other program modules and data, which have been described in greater detail herein. The mass storage device 416 may be connected to the computer 400 through a storage controller 414 connected to the chipset 406. The mass storage device 416 may consist of one or more physical storage units. The storage controller 414 may interface with the physical storage units through various type of interfaces.

The mass storage device 416 described above, the computer 400 may have access to other computer-readable storage media to store and retrieve information, such as program modules, data structures, or other data. By way of example, and not limitation, computer-readable storage media may include volatile and non-volatile, removable and non-removable media implemented in any method or technology.

The mass storage device 416 may store an operating system 430 utilized to control the operation of the computer 400. According to some configurations, the operating system may include, but is not limited to the UNIX operating system, the LINUX operating system, the WINDOWS^{®} operating system from MICROSOFT Corporation, the iOS operating system from APPLE Corporation, the ANDROID operating system from GOOGLE, and the like. Other operating systems may also be utilized. The mass storage device 416 may store other system or application programs and data utilized by the computer 400, such as components that include the data manager 432, and/or any of the other software components and data described above. The mass storage device 416 might also store other programs and data not specifically identified herein.

In one example, the mass storage device 416 or other computer-readable storage media is encoded with computer-executable instructions that, when loaded into the computer 400, implement the examples described herein. The computer 400 has access to computer-readable storage media storing computer-executable instructions which, when executed by the computer 400, may be configured to perform the various routines described above. The computer 400 might also include computer-readable storage media for performing any of the other computer-implemented operations described herein.

The mass storage device 416 may store one or more machine learning models 434 utilized to determine one or more values described herein. For example, depending on the value to be determined, the machine learning model 434 may be generated that is configured to determine the value at issue. For example, the machine learning model 434 may be configured to intake, as input, data the machine learning model 434 is configured to utilize and to perform one or more operations to determine the value. Additionally, a training dataset may be generated and utilized to train the machine learning model 434 such that a trained machine learning model 434 is generated. The trained machine learning model 434 may be utilized to determine the values described herein. Just by way of example, training of the machine learning models 434 may result in determining the one or more weighting values (e.g., associated with the health data, food data, biomarker scores, and/or user scores, etc.) health data, food data, biomarker scores, user scores (e.g., upper limit values, lower limit values, food recommendations, etc.), user data, nutritional data, other data, etc..

Predictive analytic techniques may include, for example, predictive modelling, machine learning, and/or data mining. Generally, predictive modelling may utilize statistics to predict outcomes. Machine learning, while also utilizing statistical techniques, may provide the ability to improve outcome prediction performance without being explicitly programmed to do so. A number of machine learning techniques may be employed to generate and/or modify the layers and/or models describes herein. Those techniques may include, for example, decision tree learning, association rule learning, artificial neural networks (including, in examples, deep learning), inductive logic programming, support vector machines, clustering, Bayesian networks, reinforcement learning, representation learning, similarity and metric learning, sparse dictionary learning, and/or rules-based machine learning.

Information from stored and/or accessible data may be extracted from one or more databases, and may be utilized to predict trends and behavior patterns. The predictive analytic techniques may be utilized to determine associations and/or relationships between explanatory variables and predicted variables from past occurrences and utilizing these variables to predict the unknown outcome. The predictive analytic techniques may include defining the outcome and data sets used to predict the outcome.

Data analysis may include using one or more models, including for example one or more algorithms, to inspect the data with the goal of identifying useful information and arriving at one or more determinations that assist in predicting the outcome of interest. One or more validation operations may be performed, such as using statistical analysis techniques, to validate accuracy of the models. Thereafter predictive modelling may be performed to generate accurate predictive models.

The computer 400 may also include one or more input/output controllers 410 for receiving and processing input from a number of input devices, such as an electronic data collection device, a keyboard, a mouse, a touchpad, a touch screen, an electronic stylus, or other type of input device. Similarly, the input/output controller 416 may provide output to one or more types of output devices (e.g., a display, a projector, a printer, ...). The computer 400 may not include all of the components shown in FIG. 4, may include other components that are not explicitly shown in FIG. 4, or may utilize an architecture completely different than that shown in FIG. 4.

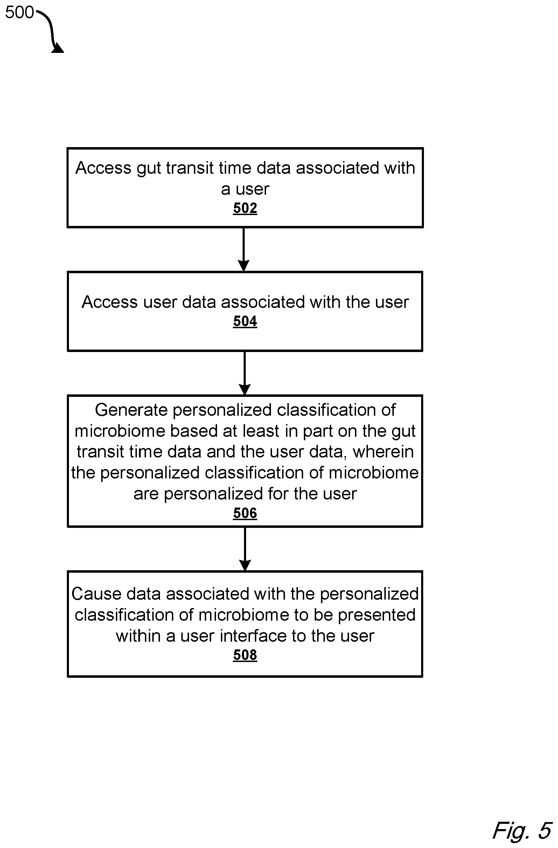

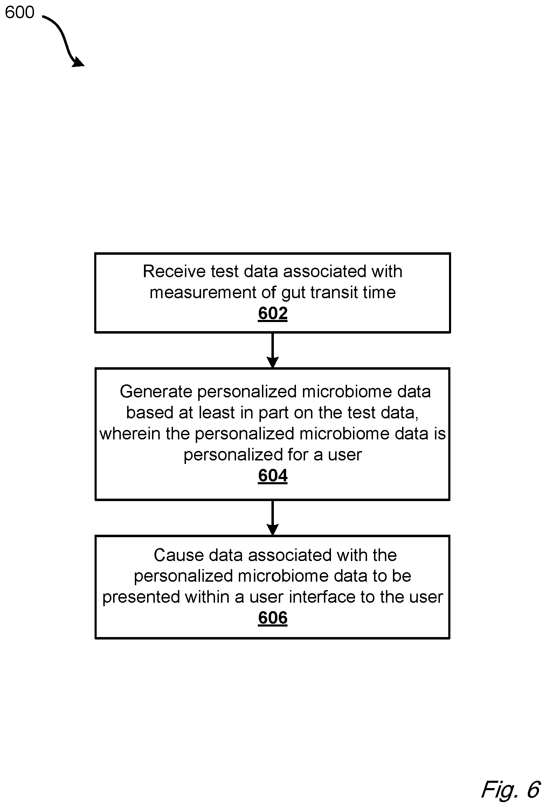

FIGS. 5 and 6 illustrate processes associated with generating predictions of gut microbiome diversity for a user. The processes described herein are illustrated as collections of blocks in logical flow diagrams, which represent a sequence of operations, some or all of which may be implemented in hardware, software or a combination thereof. In the context of software, the blocks may represent computer-executable instructions stored on one or more computer-readable media that, when executed by one or more processors, program the processors to perform the recited operations. Generally, computer-executable instructions include routines, programs, objects, components, data structures and the like that perform particular functions or implement particular data types. The order in which the blocks are described should not be construed as a limitation, unless specifically noted. Any number of the described blocks may be combined in any order and/or in parallel to implement the process, or alternative processes, and not all of the blocks need be executed. For discussion purposes, the processes are described with reference to the environments, architectures and systems described in the examples herein, such as, for example those described with respect to FIGS. 1-4, although the processes may be implemented in a wide variety of other environments, architectures and systems.

At block 502, the process 500 may include accessing gut transit time data associated with a user. For example, the UI 134 is also used when determining the gut transit time and answering questions that can be used to generate the prediction of gut microbiome diversity. For instance, the UI 134 may be used to determine a time a food was consumed that included food coloring, (or some other marker) to the time the user first saw the appearance of the color (e.g., blue) within an excreted stool. For example, the food may be injected with a blue dye (or possibly another color), which may be referred to herein as the "blue dye method" before being consumed by a user. Using the blue dye method provides benefits over other transit time measures (e.g., scintigraphy, wireless motility capsule, radiopaque markers, and breath testing) since it is inexpensive and scalable. For example, specialized equipment and staff are not required for the blue dye method. Further, gut transit time, measured via the blue dye method, is a more informative marker of gut microbiome function than traditional measures of stool consistency and frequency.

In some examples, the date and time (hours, minutes) of the appearance of the coloring (or other marker) is automatically recorded by the nutritional service 120, or some other device or component, after a user selected a UI element (e.g., clicked on the associated UI button) or some other triggering event (e.g., recognition within image data of the color of the dye within the stool). In some configurations, the user or some other device/component (e.g., image processing) may also identify/record stool frequency and/or consistency. For instance, the user, or some device/component, may record how often they have a bowel movement and record the consistency (e.g., using the Bristol Stool Form (BSF) scale). Generally, a lower BSF scale score corresponds to longer gut transit time.

At 504, the process 500 may include accessing user data associated with the user. For example, an individual (e.g., an individual associated with the computing device 132) may generate and provide user data 140A, such as health data 202 and test data 205) using a variety of at home biological collection devices, which collect a biological sample which requires a biological assay to be performed to generate the user data 140A. Some of the user data 140A may be biomarker data, such as blood glucose results collected by a continuous glucose monitor (CGM), as well as other types of data. In some cases, the other data 140B may include non-biomarker data such as photos, time stamps, as well as other data associated with a user or group of users.

At 506, the process 500 may include generating personalized classification of microbiome based at least in part on the gut transit time data and the user data, wherein the personalized classification of microbiome are personalized for the user. For example, nutritional service 120 may use the gut transit time to generate a classification for the diversity of the microbiome for the user. According to some examples, the nutritional service 120 may select from different classifications (e.g., two, three, four, ...) based on the gut transit time for the user. For instance, the nutritional service 120 may select from four different classifications (e.g., a fastest classification of less than 14 hours, a fast normal classification between 14 hours and 38 hours, a slower normal classification between 38 hours and 58 hours, and a slow classification of greater than 58 hours) depending on the gut transit time for the user. More or less classifications may be used. Gut microbiome composition predicts gut transit time classes and longer gut transit time are linked with Akkermansia muciniphila, Bacteroides and Alistipes spp relative abundances.

The gut transit time may provide a good indication of variation in the gut microbiome, in terms of both relative abundance and alpha diversity. Alpha diversity reflects how diverse a single microbiome sample is, using a measure of richness (i.e., number of species detected) and the Shannon index, which accounts for both evenness and abundance. As such, the classification for the user based on a gut transit time may be used as an informative marker of gut function and may be used to predict postprandial lipid and glucose responses and visceral fat.

At 508, the process 500 may include causing data associated with the personalized classification of microbiome to be presented within a user interface to the user. For example, the nutritional guidance service 112 provides the guidance data (e.g., the personalized health scores 108A and/or the personalized health guidance 108B) via user data 116 to a user interface display service 118 to provide to the user via UI 134. The user data 116 displayed in UI 134 may be used by an individual to help them understand how they can improve their diet. For instance, a user may receive guidance data by viewing UI 134 on a mobile device, or some other device, to determine how to improve the diversity of their gut microbiome.

FIG. 6 illustrates a flow diagram of an example process 600 for generating personalized microbiome data. The order in which the operations or steps are described is not intended to be construed as a limitation, and any number of the described operations may be combined in any order and/or in parallel to implement process 600. The operations described with respect to the process 600 are described as being performed by a nutritional service. However, it should be understood that some or all of these operations may be performed by some or all of components, devices, and/or systems described herein.

At 602, the process 600 may include receiving test data associated with measurement of gut transit time. For example, the transit time data 140E may indicate the time it takes from consuming an object to expelling the object. For instance, the gut transit time may be the time between ingestion of a food including food coloring paste, such as royal blue (or some other color), and the first time the user sees the appearance of the color of the food coloring in their stool. According to some configurations, a user eats food, such as a muffin, that has been died "blue," such that it is easily identified by its color when it appears in the stool of the user.

According to some configurations, a nutritional service 120 may utilize the transit time data 140E to generate the prediction of the gut microbiome diversity for a user. For example, the nutritional service 120 may use a machine learning mechanism to perform an analysis of the transit time data 140E to generate the prediction. In some examples, the nutritional service 120 identifies a uniqueness of the microbiome, identify interesting species, and the like. According to some configurations, the microbiome data may include gut transit time.

At 604, the process 600 may include generating personalized microbiome data based at least in part on the test data, wherein the personalized microbiome data is personalized for the user. For example, nutritional service 120 may use the gut transit time to generate a classification for the diversity of the microbiome for the user. According to some examples, the nutritional service 120 may select from different classifications (e.g., two, three, four, ...) based on the gut transit time for the user. For instance, the nutritional service 120 may select from four different classifications (e.g., a fastest classification of less than 14 hours, a fast normal classification between 14 hours and 38 hours, a slower normal classification between 38 hours and 58 hours, and a slow classification of greater than 58 hours) depending on the gut transit time for the user. More or less classifications may be used. Gut microbiome composition predicts gut transit time classes and longer gut transit time are linked with Akkermansia muciniphila, Bacteroides and Alistipes spp relative abundances.

The gut transit time may provide a good indication of variation in the gut microbiome, in terms of both relative abundance and alpha diversity. Alpha diversity reflects how diverse a single microbiome sample is, using a measure of richness (i.e., number of species detected) and the Shannon index, which accounts for both evenness and abundance. As such, the classification for the user based on a gut transit time may be used as an informative marker of gut function and may be used to predict postprandial lipid and glucose responses and visceral fat.

At 606, the process 600 may include causing data associated with the personalized microbiome data to be presented within a user interface to the user. For example, the nutritional guidance service 112 provides the guidance data (e.g., the personalized health scores 108A and/or the personalized health guidance 108B) via user data 116 to a user interface display service 118 to provide to the user via UI 134. The user data 116 displayed in UI 134 may be used by an individual to help them understand how they can improve their diet. For instance, a user may receive guidance data by viewing UI 134 on a mobile device, or some other device, to determine how to improve the diversity of their gut microbiome.

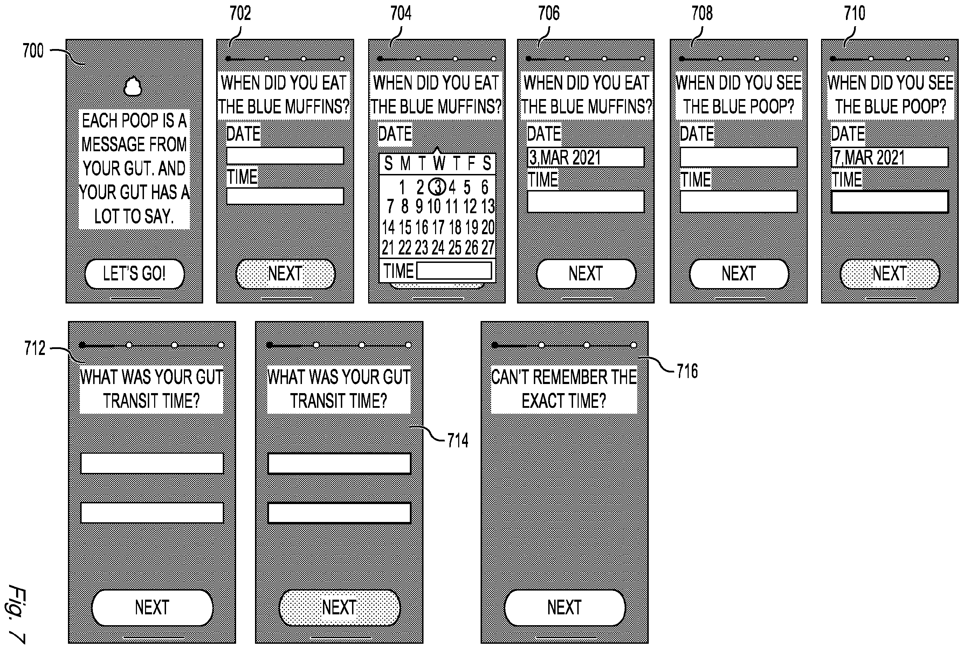

FIGS. 7-24 illustrate example user interfaces that may be displayed to a user who is tracking their gut transit time via the nutritional service 120.

FIG. 7 illustrates examples, user interfaces (UIs) 700-716 for entering time data associated with recording a transit time of a consumed food item. For example, UI 700 may be an initial startup page enabling a user to initiate the gut transit tracking time process. UI 702 may enable a user to enter a day and a time indicating when the food item was ingested. UI 704 may enable a user to select a day and time. from a displayed calendar, in which the food item was ingested. UI 706 may enable a user to submit the day and time in which the food item was ingested. UI 708 may enable a user to enter a day and time in which the food item was expelled. UI 710 may enable a user to submit the day and time when the food item was expelled. UI 712 may enable a user to enter a gut transit time calculated by a user. UI 714 may enable a user to indicate that they do not remember the gut transit time. UI 716 may enable a user to submit that they do not remember the gut transit time.

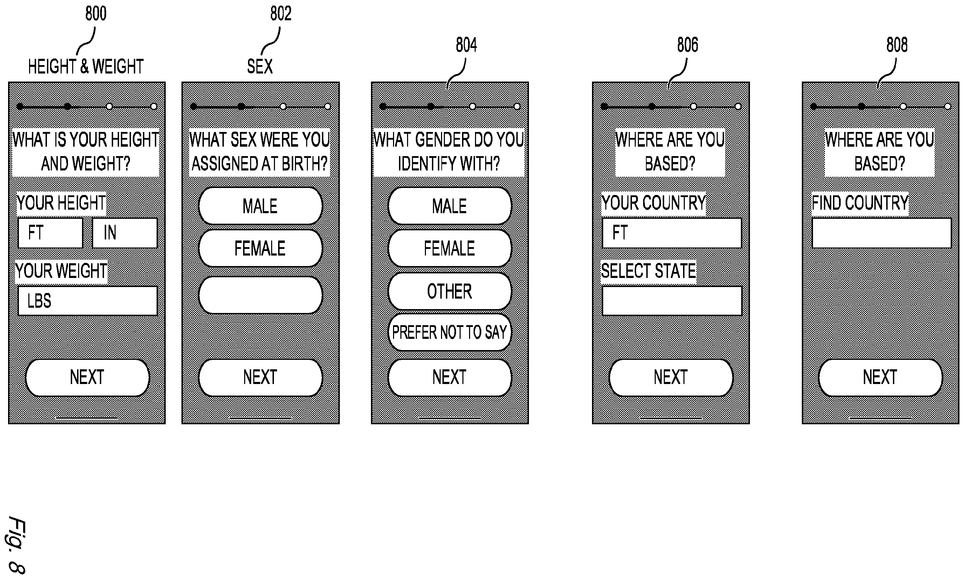

FIG. 8 illustrates example UIs 800-808 for receiving user data associated with the user. For example, UI 800 may enable a user to enter and/or submit information regarding their height and/or weight. UI 802 may enable a user to enter and/or submit their assigned gender at birth. UI 804 may enable a user to enter and/or submit their currently identified gender. UIs 806 and 808 may enable a user to enter and/or submit location information, such has where the user currently lives and/or where the user has previously lived.

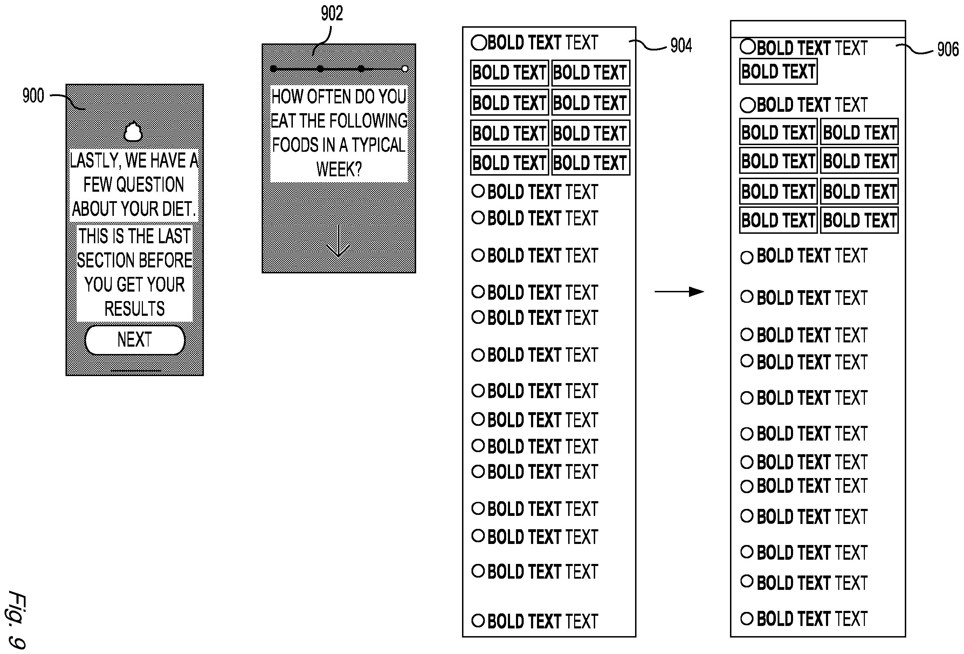

FIG. 9 illustrates example UIs 900-906 for receiving food data associated with food items that the user consumes. For example, UIs 900 and 902 may inform the user that they are going to provide food consumption data on the following UIs and instruct the user to select which food items they consume. UIs 904 and 906 may enable the user to select food items from a list of food items and indicate how often they eat those food items.

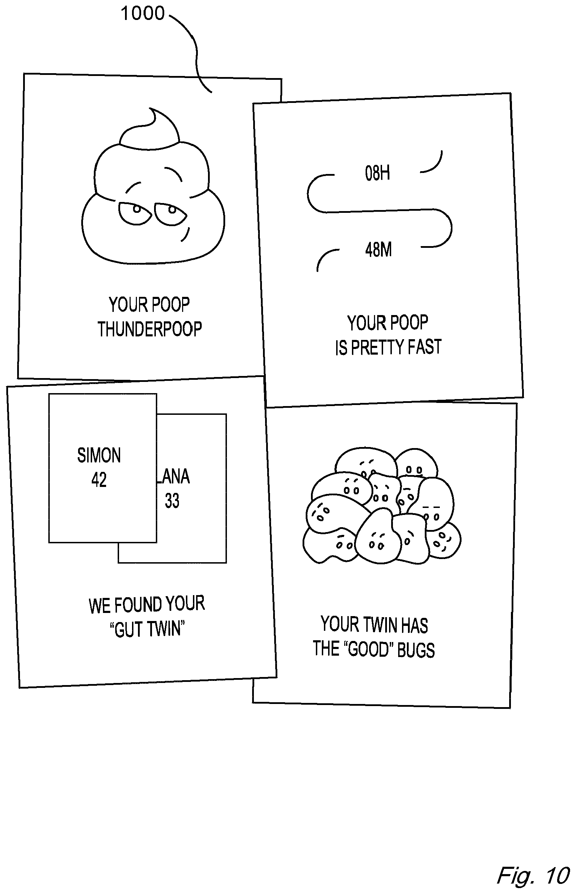

FIG. 10 illustrates an example UI 1000 for presenting information to the user that classifies the gut transit time of the user (e.g., "thunderpoop"), a gut transit time, an indication of how the gut transit time compares to other users, a "gut twin" that indicates a similar user based on the predicted diversity of gut microbiome, and microbe information.

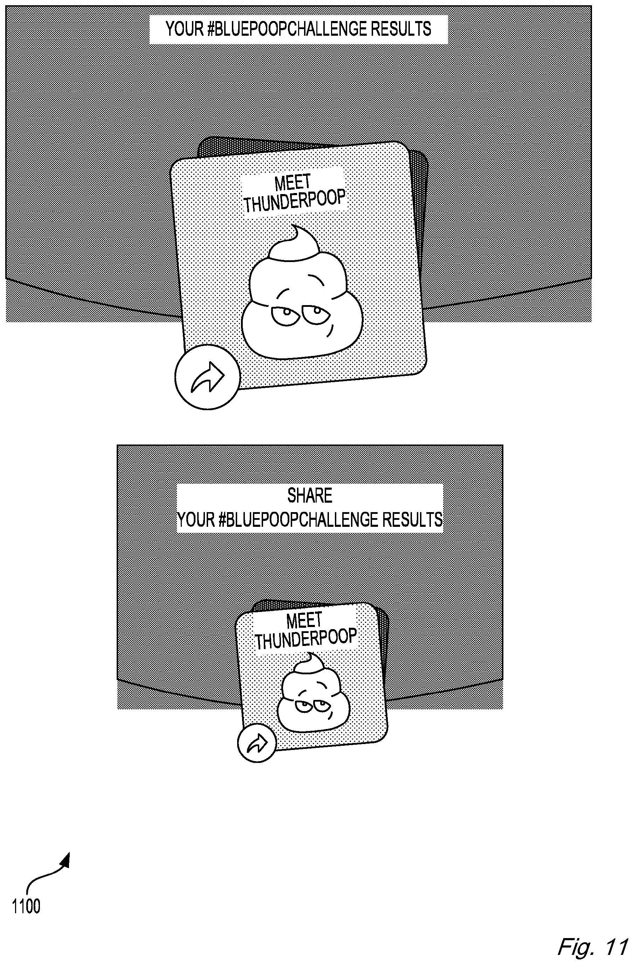

FIG. 11 illustrates example UIs 1100 for presenting information to the user that classifies the gut transit time of the user (e.g., "thunderpoop"). In some cases, the UIs 1100 may include recommendations and/or selectable icons to share results of the gut transit time via one or more social media sites.

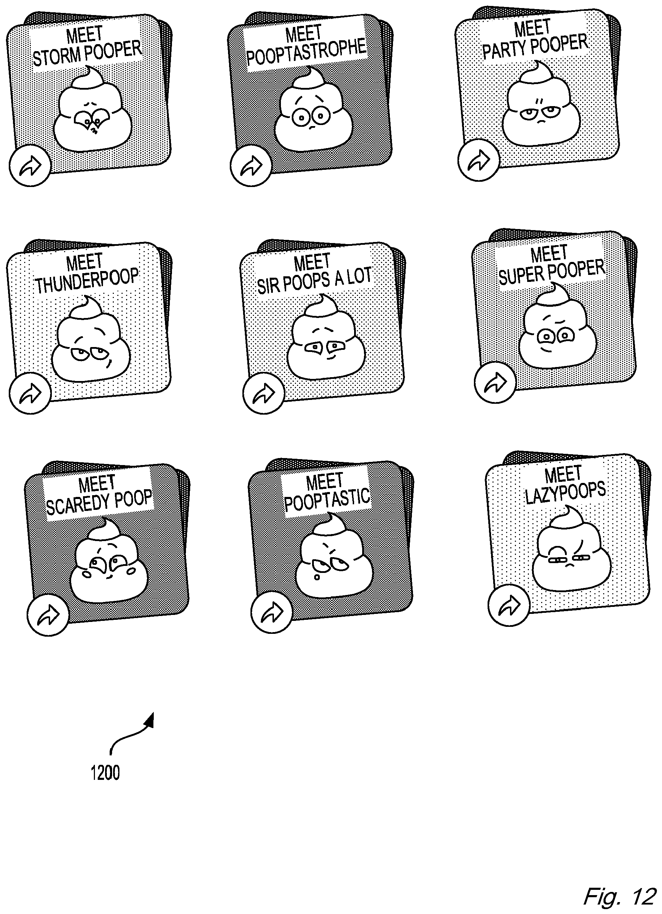

FIG. 12 illustrates example UIs 1200 for presenting a variety of information to the user that includes multiple classifiers of the gut transit time of the user. In some examples, the classifiers may include "storm pooper," "pooptastrophe," "party pooper," "thunderpoop," "sir poops a lot," "super pooper," "scaredy poop," "poopstastic," and/or "lazypoops."

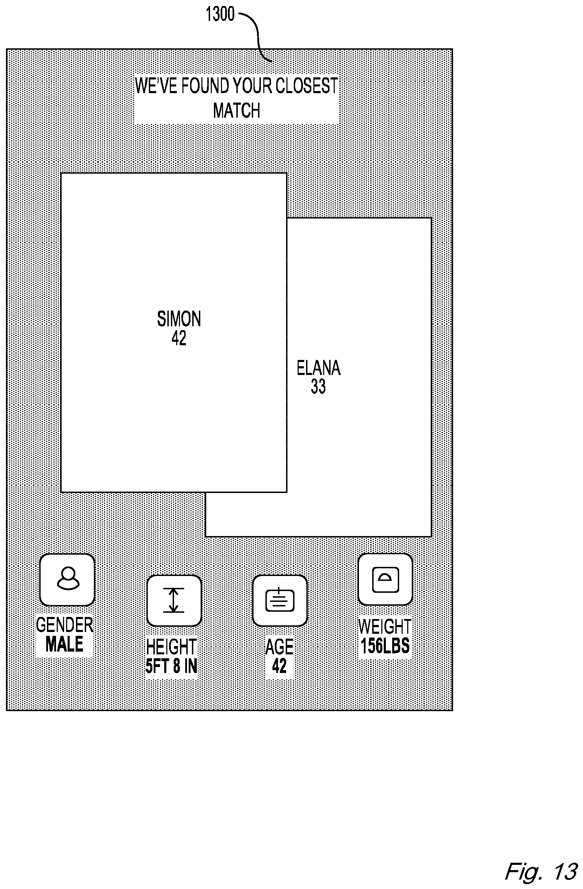

FIG. 13 illustrates an example UI 1300 for presenting information to the user that classifies a "gut twin" that indicates a similar user based on the predicted diversity of gut microbiome. In some cases the UI 1300 may include the user data that the "gut twin" shares with the user (e.g., gender, height, age, weight, etc.).

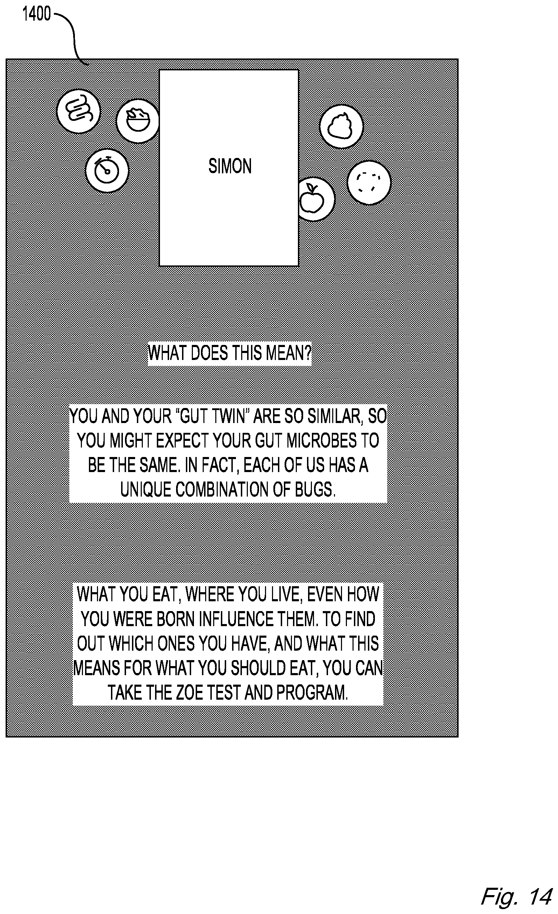

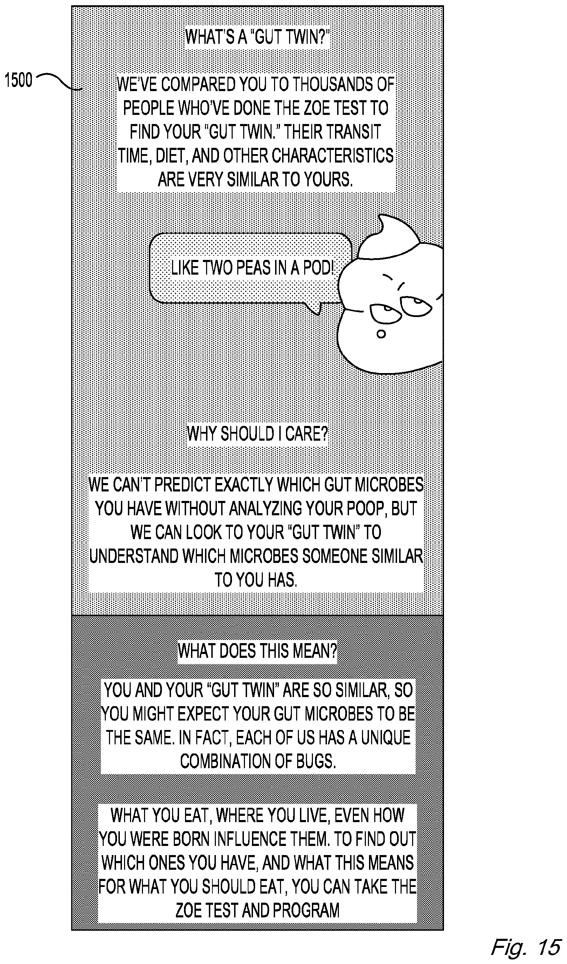

FIGS. 14 and 15 illustrate example UIs 1400 for and 1500 providing additional information regarding what having a "gut twin" is associated with (e.g., both users may have similar gut microbiome diversity) and why it is important. In some cases, the UIS 1400 and 1500 may include the user data that the "gut twin" shares with the user (e.g., gender, height, age, weight, etc.).

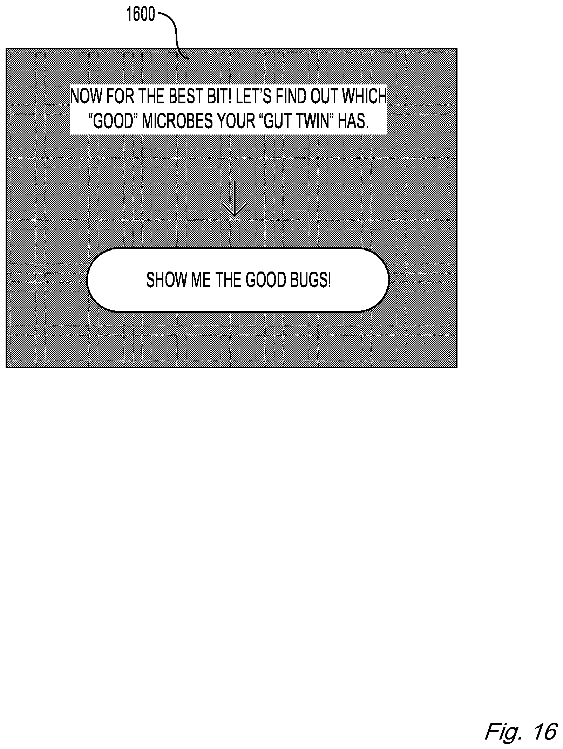

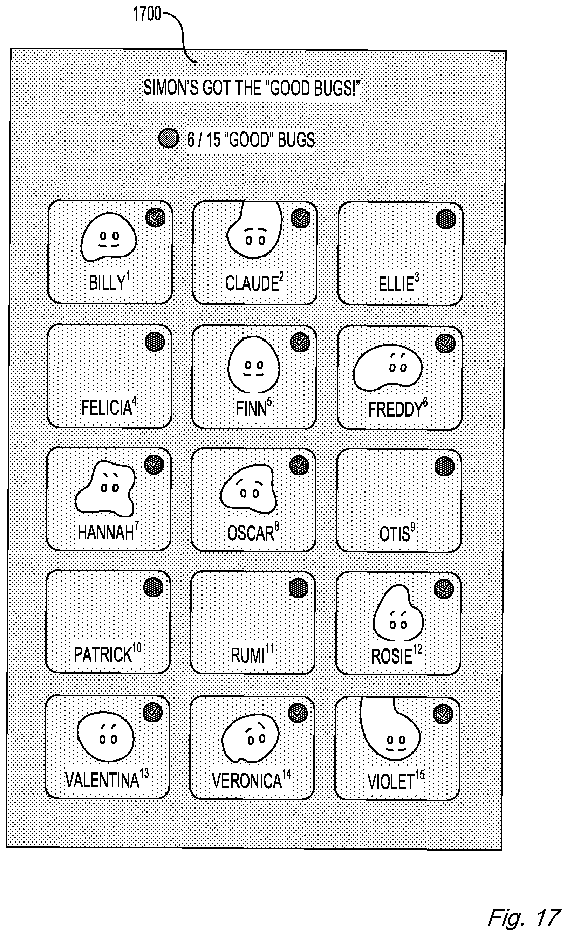

FIGS. 16 and 17 illustrates an example UI 1600 and UI 1700 for enabling a user to make a selection to present the "good" microbiome present in their gut. In some cases, the UI 1700 may enable a user to view the "good" microbiome that may be present (e.g., "Billy," "Claude," "Finn" etc.) in their gut as well as a number of other types of microbiome that may not be present (e.g., "Felicia," Otis," "Finn" etc.).

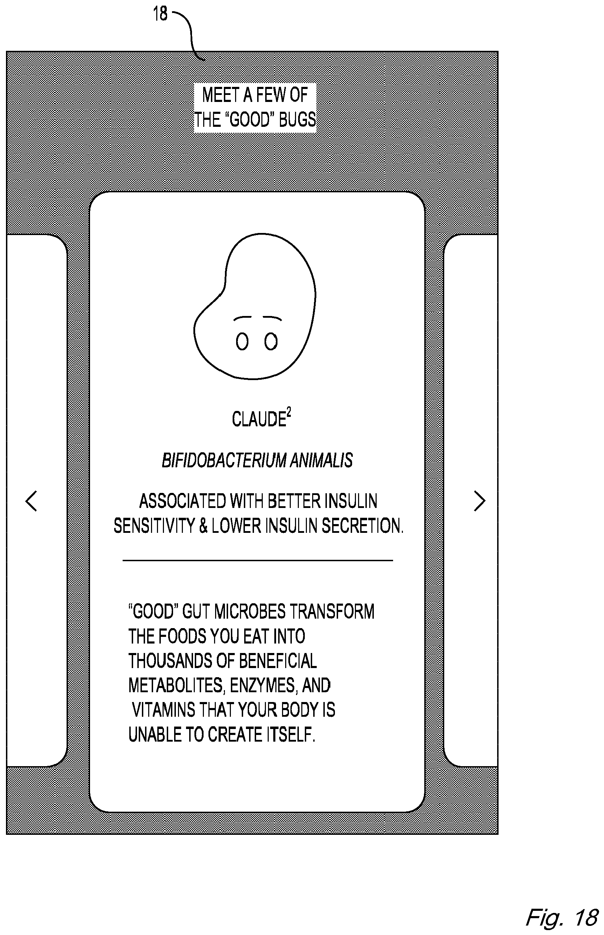

FIG. 18 illustrates an example UI 1800 for enabling the user to view detailed information regarding a specific microbiome (e.g., "Claude" which may be a Bifidobacterium animalis). In some examples, the UI 1800 may include additional information about a "good" microbiome, such as how microbes transform food into metabolites, enzymes and vitamins.

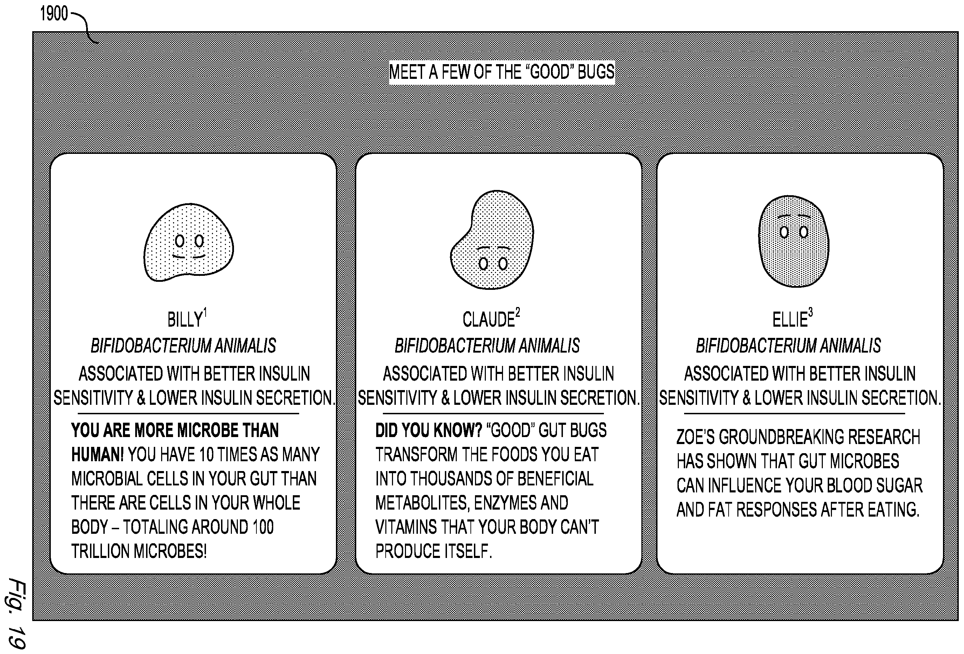

FIG. 19 illustrates an example UI 1900 for enabling the user to view detailed information regarding multiple microbiome (e.g., "Billy," "Claude," "Ellie" etc.). In some examples, the UI 1900 may include additional information about a "good" microbiome, such as influencing blood sugar and fat.

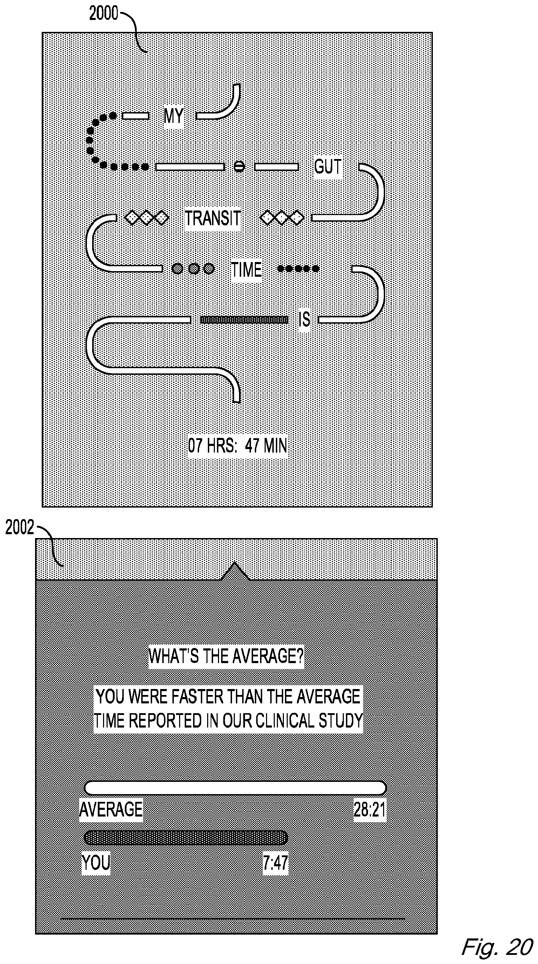

FIG. 20 illustrates an example UIs 2000 and 2002 for enabling a user to view their gut transit time (e.g., 7hr. 4min.) as well as their gut transit time relative to an average gut transit of a group of other users (e.g., 28hr. 21min. ). In some cases, the gut transit time may be illustrated via a line or bar diagram. In some cases, the transit time may be presented via numerical values.

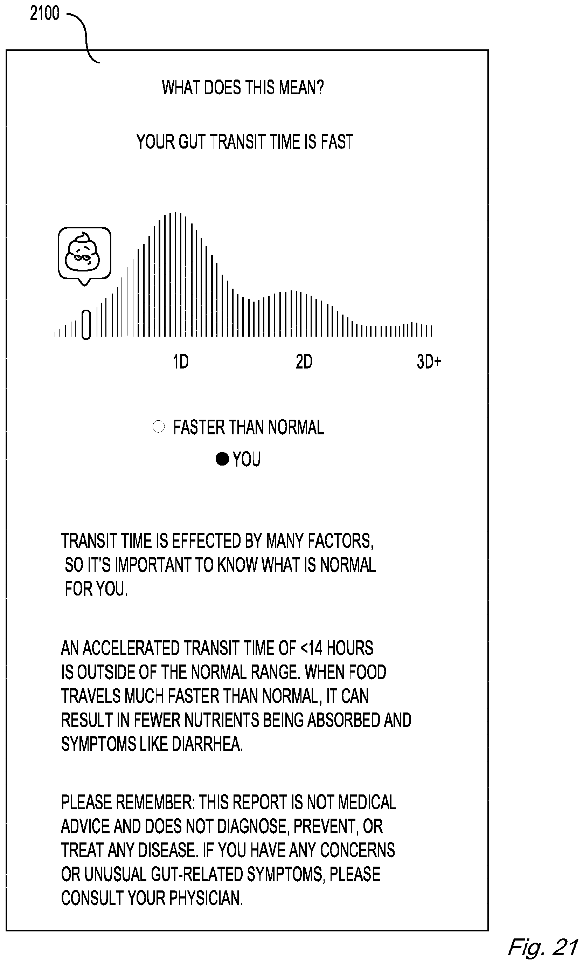

FIG. 21 illustrates an example UI 2100 for enabling a user to view additional information regarding what their gut transit time means relative to the average gut transit time (e.g., shorter than average gut transit time may indicate that your body is absorbing less nutrients from the food item). In some cases, the UI 2100 may include a disclaimer informing the user that the report is not medical advice or a diagnosis of a disease.

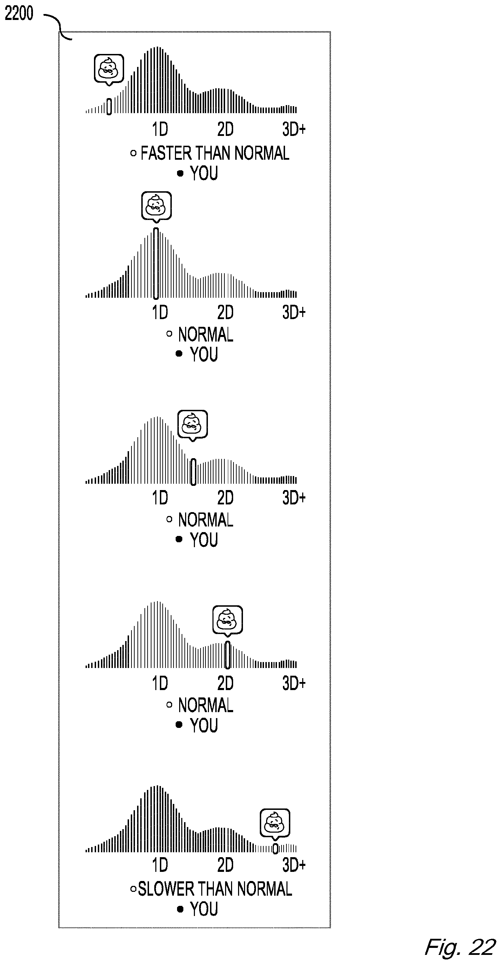

FIG. 22 illustrates an example UI 2200 for enabling a user to view a number of different curves showing the users gut transit time relative to other users gut transit times (e.g., "faster than normal," "normal," "slower than normal," etc.). In some cases, the UI 2200 may illustrate the curves as bell curves.

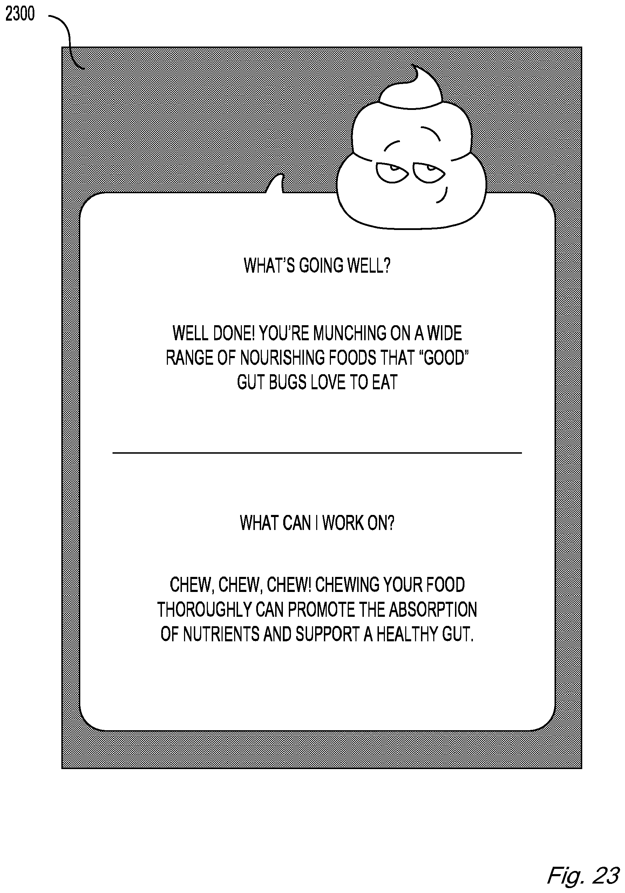

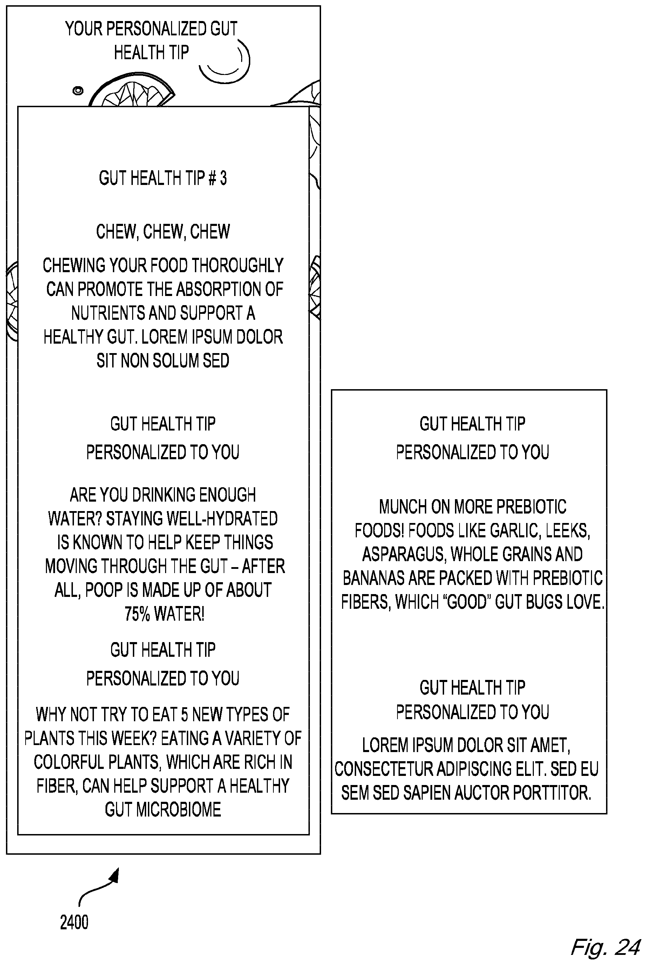

FIGS. 23 and 24 illustrate example UI 2300 and UI 2400 for enabling a user to view positive aspects associated with their test data (e.g., good variety of food) and aspects associated with their test data that needs improvement (e.g., an indication of improved chewing). In some cases, UI 2400 may enable a user to view personalized health tips based on their test data (e.g., chew more, hydrate more, eat more prebiotic food, etc.).

Based on the foregoing, it should be appreciated that technologies for generating a prediction gut biome diversity of a user have been presented herein. Moreover, although some of the subject matter presented herein has been described in language specific to computer structural features, methodological acts and computer readable media, it is to be understood that the invention defined in the appended claims is not necessarily limited to the specific features, acts, or media described herein. Rather, the specific features, acts and media are disclosed as example forms of implementing at least some of the claims.

The subject matter described above is provided by way of illustration only and should not be construed as limiting. Furthermore, the claimed subject matter is not limited to implementations that solve any or all disadvantages noted in any part of this disclosure. Various modifications and changes may be made to the subject matter described herein without following the examples and applications illustrated and described, and without departing from the true spirit and scope of the present invention, which is set forth in the following claims.

### Example clauses

A: A method, comprising accessing gut transit time data associated with a user, accessing user data associated with the user, generating personalized classification of microbiome based at least in part on the gut transit time data and the user data, wherein the personalized classification of microbiome are personalized for the user, and causing data associated with the personalized classification of microbiome to be presented within a user interface to the user.
B: The method of clause A, wherein the user comprises a first user, the gut transit time data comprises first gut transit time data, and the user data comprises first user data, the method further comprising: identifying a second user based at least in part on one of: a characteristic of the first user gut transit time data, first user data, an equivalent characteristic of second gut transit time data associated with the second user, or second user data associated with the second user being similar to the first user data, accessing microbiome data associated with the second user; and generating the personalized classification of microbiome based at least in part on the microbiome data associated with the second user.
C: The method of clause A, wherein the gut transit time data is received via an application stored on a device associated with the user, the gut transit time data indicating an amount of gut transit time it takes from consuming an object to expelling the object.
D: The method of clause A, wherein the amount of gut transit time is associated with a classification based at least in part on a distribution of gut transit times in a population.
E: The method of clause A, wherein the user data includes at least one of age, height, weight, sex assigned at birth, gender identification, location information, diet information, stool information, pet information, or allergy information.
F: The method of clause A, further comprising accessing nutrition data associated with the user, and generating the personalized classification of microbiome based at least in part on the nutrition data.
G: The method of clause F, wherein the nutrition data is associated with at least one previously eaten food item and includes at least one of a frequency of consumption, a quantity of consumption, grams of fiber, level of food item processing, type of fiber, grams of carbohydrate, glycemic index, sugar content, salt content, alcohol content, whether the food item is fermented, whether the food item is a high polyphenol food item, whether the food item is meat, the composition and source of fat in the food item, a composition of fiber in the food item, a production method, preparation methods applied by the user, an inclusion of flavor enhancers, an inclusion of emulsifiers, a method by which the food item was preserved, a location where the food item was produced, a method by which animals contributing to the food item were reared, a method by which animals contributing to the food item were caught, or a method by which animals contributing to the food item were slaughtered.
H: The method of clause A, further comprising receiving at least one input identifying at least one food item, determining food data associated with the at least one food item, determining health data associated with the user based at least in part on the gut transit time data, generating a postprandial response prediction associated with the at least one food item based at least in part on the food data and the health data, and associating at least one biomarker score with the at least one food item based at least in part on the postprandial response prediction.
I: The method of clause A, further comprising generating, based at least in part on the gut transit time data and the user data, a recommendation to the user for improving microbiome diversity, increasing good microbes, or decreasing bad microbes, and causing data associated with the recommendation to be presented within the user interface to the user.
J: The method of clause I, wherein the recommendation includes at least one of an eating habit recommendation, a hydration recommendation, or a food item consumption recommendation.
K: A system comprising one or more processors, and one or more non-transitory computer-readable media comprising instructions that, when executed by the one or more processors, cause the one or more processors to perform operations comprising accessing gut transit time data associated with a user, accessing user data associated with the user, generating personalized classification of microbiome based at least in part on the gut transit time data and the user data, wherein the personalized classification of microbiome are personalized for the user, and causing data associated with the personalized classification of microbiome to be presented within a user interface to the user.
L: The system of clause K, further comprising receiving additional gut transit time data associated with additional users, determining an average gut transit time data associated with the additional gut transit time data, and causing data associated with a comparison of the average gut transit time data to the gut transit time data associated with the user to be presented within the user interface to the user.
M: The system of clause K, wherein the user comprises a first user, the gut transit time data comprises first gut transit time data, and the user data comprises first user data, the method further comprising: identifying a second user based at least in part on one of: a characteristic of the first user gut transit time data, first user data, an equivalent characteristic of second gut transit time data associated with the second user, or second user data associated with the second user being similar to the first user data, accessing microbiome data associated with the second user; and generating the personalized classification of microbiome based at least in part on the microbiome data associated with the second user.
N: The system of clause K, wherein the gut transit time data is received via an application stored on a device associated with the user, the gut transit time data indicating an amount of gut transit time it takes from consuming an object to expelling the object.
O: The system of clause N, wherein the amount of gut transit time is associated with a classification based at least in part on a distribution of gut transit times in a population.
P: A method comprising, receiving test data associated with measurement of gut transit time, generating personalized microbiome data based at least in part on the test data, wherein the personalized microbiome data is personalized for the user, and causing data associated with the personalized microbiome data to be presented within a user interface to the user.
Q: The method of clause P, wherein the personalized microbiome data is generated by a machine learning model.
R: The method of clause P, wherein the personalized microbiome data includes at least a classification of at least one microbe located within the user, the at least one microbe including at least one of Akkermansia muciniphila, Bacteroides or Alistipes spp.
S: The method of clause P, further comprising generating personalized microbiome data based on accessing at least one of a family health history associated with the user, measures of blood chemistry taken in a fasting state associated with the user, or measures of blood chemistry taken in a postprandial state associated with the user.
T: The method of clause P, further comprising generating a quality score associated with the personalized microbiome data indicating an accuracy level of the personalized microbiome data.

## Claims

1. A method, comprising:
accessing gut transit time data associated with a user;
accessing user data associated with the user;
generating personalized classification of microbiome based at least in part on the gut transit time data and the user data, wherein the personalized classification of microbiome are personalized for the user; and
causing data associated with the personalized classification of microbiome to be presented within a user interface to the user.

2. The method of claim 1, wherein the user comprises a first user, the gut transit time data comprises first gut transit time data, and the user data comprises first user data, the method further comprising:
identifying a second user based at least in part on one of:
a characteristic of the first user gut transit time data;
first user data,
an equivalent characteristic of second gut transit time data associated with the second user; or
second user data associated with the second user is within a threshold time to the first user data;
accessing microbiome data associated with the second user; and
generating the personalized classification of microbiome based at least in part on the microbiome data associated with the second user.

3. The method of claim 1 or 2, wherein the gut transit time data is received via an application stored on a device associated with the user, the gut transit time data indicating an amount of gut transit time it takes from consuming an object to expelling the object.

4. The method of claim 3, wherein the amount of gut transit time is associated with a classification based at least in part on a distribution of gut transit times in a population.

5. The method of any of the preceding claims, wherein the user data includes at least one of age, height, weight, sex assigned at birth, gender identification, location information, diet information, stool information, pet information, or allergy information.

6. The method of any of the preceding claims, further comprising:
accessing nutrition data associated with the user; and
generating the personalized classification of microbiome based at least in part on the nutrition data.

7. The method of claim 6, wherein the nutrition data is associated with at least one previously eaten food item and includes at least one of a frequency of consumption, a quantity of consumption, grams of fiber, level of food item processing, type of fiber, grams of carbohydrate, glycemic index, sugar content, salt content, alcohol content, whether the food item is fermented, whether the food item is a high polyphenol food item, whether the food item is meat, the composition and source of fat in the food item, a composition of fiber in the food item, a production method, preparation methods applied by the user, an inclusion of flavor enhancers, an inclusion of emulsifiers, a method by which the food item was preserved, a location where the food item was produced, a method by which animals contributing to the food item were reared, a method by which animals contributing to the food item were caught, or a method by which animals contributing to the food item were slaughtered.

8. The method of any of the preceding claims, further comprising:
receiving at least one input identifying at least one food item;
determining food data associated with the at least one food item;
determining health data associated with the user based at least in part on the gut transit time data;
generating a postprandial response prediction associated with the at least one food item based at least in part on the food data and the health data; and
associating at least one biomarker score with the at least one food item based at least in part on the postprandial response prediction.

9. The method of any of the preceding claims, further comprising:
generating, based at least in part on the gut transit time data and the user data, a recommendation to the user for improving microbiome diversity, increasing good microbes, or decreasing bad microbes; and
causing data associated with the recommendation to be presented within the user interface to the user.

10. The method of claim 9, wherein the recommendation includes at least one of an eating habit recommendation, a hydration recommendation, or a food item consumption recommendation.

11. The method of any of the preceding claims, further comprising:
receiving additional gut transit time data associated with additional users;
determining an average gut transit time data associated with the additional gut transit time data; and
causing data associated with a comparison of the average gut transit time data to the gut transit time data associated with the user to be presented within the user interface to the user.

12. A system comprising:
one or more processors; and
a computer program comprising instructions that, when executed by the one or more processors, cause the one or more processors to perform the method of any of the preceding claims.

13. A method comprising:
receiving test data associated with measurement of gut transit time;
generating personalized microbiome data based at least in part on the test data, wherein the personalized microbiome data is personalized for a user; and
causing data associated with the personalized microbiome data to be presented within a user interface to the user.

14. The method of claim 13, wherein the personalized microbiome data is generated by a machine learning model.

15. The method of claim 13 or 14, wherein the personalized microbiome data includes at least a classification of at least one microbe located within the user, the at least one microbe including at least one of Akkermansia muciniphila, Bacteroides or Alistipes spp.
